(19) Europäisches Patentamt
European Patent Office
Office européen des brevets

(11) Veröffentlichungsnummer: **0 137 499**
**A2**

(12) # EUROPÄISCHE PATENTANMELDUNG

(21) Anmeldenummer: 84112192.4

(22) Anmeldetag: 11.10.84

(51) Int. Cl.⁴: C 07 J 9/00
A 61 K 31/575

(30) Priorität: 13.10.83 DE 3337229

(43) Veröffentlichungstag der Anmeldung:
17.04.85 Patentblatt 85/16

(84) Benannte Vertragsstaaten:
AT BE CH DE FR GB IT LI LU NL SE

(71) Anmelder: A. Nattermann & Cie. GmbH
Nattermannallee 1
D-5000 Köln 30(DE)

(72) Erfinder: Stapel, Günther, Dr.
Münstereifeler Strasse 30
D-5000 Köln 41(DE)

(72) Erfinder: Gober, Karl-Heinz, Dr. Dipl.-Chem.
Archimedesstrasse 38
D-5000 Köln 80(DE)

(72) Erfinder: Leyck, Sigurd, Dr. Dipl.-Bio.
Am Quechenhauf 21
D-5024 Pulheim 2(DE)

(72) Erfinder: Wetzig, Helmut, Dr.
Paulstrasse 46
D-5024 Pulheim-Sinnersdorf(DE)

(72) Erfinder: Birkner, Christian, Dr. Dipl.-Chem.
Grubenbecherstrasse
D-5000 Köln 30(DE)

(72) Erfinder: Kesselring, Kurt, Dr.
Villenweg 49
D-5024 Erftstadt-Köttingen(DE)

(72) Erfinder: Christ, Bruno, Dr.
Peter-Franzen-Strasse 44
D-5000 Köln 30(DE)

(72) Erfinder: Steiner, Klaus, Dr. Dipl.-Chem.
Gartenstrasse 18
D-5024 Pulheim-Stommeln(DE)

(72) Erfinder: Schröder, Friedhelm
An der Maar 10
D-5024 Pulheim 2(DE)

(74) Vertreter: Sternagel, Hans-Günther, Dr. et al,
Patentanwälte Dr. M. Hann Dr. H.-G. Sternagel
Marburger Strasse 38
D-6300 Giessen(DE)

(54) **Verfahren zur Gewinnung von beta-Elemonsäure und diese enthaltende pharmazeutische Zubereitungen.**

(57) Die vorliegende Erfindung betrifft die Gewinnung der ß-Elemonsäure aus Elemiharz und pharmazeutische Zubereitungen, die als Wirkstoff ß-Elemonsäure enthalten.

Die pharmazeutischen Zubereitungen zeigen entzündungshemmende Eigenschaften und eignen sich besonders zur Behandlung von entzündlichen Krankheitsprozessen bei Mensch oder Tier.

EP 0 137 499 A2

Croydon Printing Company Ltd.

Anmelder: A. Nattermann & Cie GmbH (E-1653)
Nattermannallee 1, 5000 Köln 30

Titel: Verfahren zur Gewinnung von ß-Elemonsäure und diese enthaltende pharmazeutische Zubereitungen.

Beschreibung

Die Erfindung betrifft ein Verfahren zur Gewinnung von ß-Elemonsäure und pharmazeutischen Zubereitungen für die Human- und Veterinärtherapie, welche als wirksame Komponente ß-Elemonsäure enthalten, insbesondere zur Behandlung von entzündlichen Prozessen.

ß-Elemonsäure (13α, 14ß, 17α, 20S, 3-Oxo-lanosta-8,24-dien-21-carbonsäure)

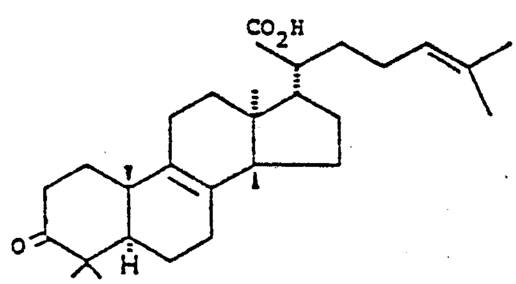

ist ein bekanntes Triterpen, welches im sogenannten Elemiharz vorkommt. Elemiharz wird aus verschiedenen Burserazeen, besonders aus der Gattung Canaricum, gewonnen (Berger, Bd. 6, S. 97-99; Hagers Handbuch der pharm. Praxis Bd. IV, S. 765-766). Das Elemiharz enthält ca. 20 - 30 % etherische Öle, ca. 20 - 25 % Amyrine, 5 - 6 % Elemisäuren und verschiedene Sesquiterpenalkohole und andere Triterpensäuren.

Elemiharz wurde in der Volksmedizin zur Abdeckung von bösartigen Wunden eingesetzt oder auch als Pflastergrundlage zur besseren Haftung verwendet. Das Harz wird weiterhin in der Industrie zur Herstellung von Lacken und Firnissen und zum Appretieren von Filzgewebe benötigt.

Zur Aufklärung der Struktur der Triterpensäuren aus Elemiharz wurden z. B. von L. Ruzicka und H. Häusermann, Helv. Chim. Acta, 1942, 25, 439-57, die einzelnen Substanzen isoliert.
ß-Elemonsäure, die nur zu ca. 2 % im Elemiharz vorkommt, wurde bisher noch nicht therapeutisch eingesetzt, auch pharmakologische Untersuchungen sind bisher nicht bekannt.

Aufgabe der Erfindung ist ein besonders vorteilhaftes Verfahren zur Gewinnung von ß-Elemonsäure aus Pflanzen bzw. pflanzlichen Harzen zur Verwendung in pharmazeutischen Zubereitungen.
Diese Aufgabe wird durch das Verfahren gemäß Patentanspruch 1 gelöst.

Die Erfindung schließt auch pharmazeutische Zubereitungen ein, die als aktiven Wirkstoff ß-Elemonsäure enthalten.
Es wurde nämlich überraschend gefunden, daß die reine ß-Elemonsäure eine starke entzündungshemmende Wirkung besitzt.
ß-Elemonsäure eignet sich daher besonders zur Therapie von rheumatischen Krankheiten, wie z.B. Arthrosen oder chronischer Polyarthritis, wobei sie sich durch eine sehr gute Verträglichkeit auszeichnet.

Nach den bisher bekannten Verfahren zur Isolierung von ß-Elemonsäure aus Elemiharzen, insbesondere Manila Elemi, wird das Harz in einem organischen, mit Wasser nicht mischbaren Lösungsmittel, wie z.B. Dialkylether, Alkylcarbonsäureester, Kohlenwasserstoffen, halogenisierten Kohlenwasserstoffen oder Aromaten, wie z.B. Toluol, gelöst und anschließend mit einer wäßrigen, alkalischen Lösungs, insbesondere 0,5N-Natronlauge, intensiv durchmischt. Die wäßrige, alkalische Phase wird abgetrennt und mit 20 - 30 %iger Salzsäure unter Rühren auf pH 3 angesäuert und anschließend mit einem organischen Lösungsmittel, wie z.B. Toluol, ausgeschüttelt und die organische Phase getrocknet. Nach dem Eindampfen des Lösungsmittels wird ein Gemisch von Elemisäuren erhalten (z.B. L. Ruzicka, J.R. Hosking und A.Wick, Helv. Chim. Acta <u>14</u> (1931), 811-820). Aus diesem Gemisch wird die ß-Elemonsäure durch Umsetzung mit Girard-T-Reagenz in ein Hydrazon überführt und durch Ausschütteln mit Wasser von den anderen Elemisäuren abgetrennt. Nach Freisetzung durch Ansäuern kann die ß-Elemonsäure extrahiert und rein isoliert werden (z.B. L.Ruzicka und H.Häusermann, Helv. Chim. Acta <u>25</u> (1942), 439-57).

Manila-Elemi-Harz enthält durchschnittlich

- 3 - 5 % ß-Elemonsäure
- 8 - 10 % $\Delta^8$-α- Elemolsäure
- 4 - 6 % $\Delta^7$-α- Elemolsäure

Nach den bisher beschriebenen Methoden lassen sich nur die 3 - 5 % ß-Elemonsäuren zur Isolierung nutzen. Um die im Harz vorhandene $\Delta^8$-α-Elemolsäure durch Oxidation in die ß-Elemonsäure zu überführen, bedarf es weiterer aufwendiger Reinigungsschritte.

Es wurde nun ein Verfahren gefunden, bei dem unter Ausnutzung aller im Elemisäure-Konzentrat vorhandenen Elemisäuren auf einem einfachen kurzen Weg ß-Elemonsäure gewonnen wird. Dabei wird zur Gewinnung eines Elemisäurekonzentrates das Elemiharz in einem alkoholischen Lösungsmittel, wie z.B. Methanol, aufgeschlämmt und der Rückstand abfiltriert.

Die klare Lösung wird mit einem basischen Ionenaustauscher verrührt. Anschließend wird das Austauscherharz abfiltriert, mit einem alkoholischen Lösungsmittel ausgewaschen, danach mit methanolischer Essigsäurelösung eluiert und das Eluat eingedampft. Im Rückstand befinden sich die Elemisäuren, ß-Elemonsäure, $\Delta^7$-α-Elemolsäure und $\Delta^8$-α-Elemolsäure.
Der Vorteil dieses Verfahrens liegt in der annähernd quantitavien Extraktionsausbeute bei einem Gehalt von 90 - 95 % Elemisäuren im Konzentrat. Ein weiterer Vorteil ist die Wiederverwendbarkeit der Ionenaustauscher nach Regeneration unter Abtrennung von ca. 20 % unlöslicher Nebenprodukte, besonders Amyrine. Die Isomerisierung der im Elemisäurekonzentrat enthaltenen $\Delta^7$-α-Elemolsäure zur $\Delta^8$-α-Elemolsäure wird ohne vorherige Auftrennung der Elemisäuren in einem chlorierten Kohlenwasserstoff unter Verwendung einer Mineralsäure durchgeführt.
Die Oxydation von $\Delta^8$-α-Elemolsäure zur ß-Elemonsäure wird im rohen Gemisch durch Oppenauer-Reaktion mit Aluminiumisopropylat in Cyclohexanon durchgeführt. Die Endreinigung erfolgt durch einfache Säulenchromatographie an Kieselgel. Die Ausbeuten von ß-Elemonsäure aus Manila-Elemi-Harz können so wesentlich verbessert werden. Die Überlegenheit des neuen Verfahrens wird durch das Schema der Gewinnung von ß-Elemonsäure verdeutlicht.

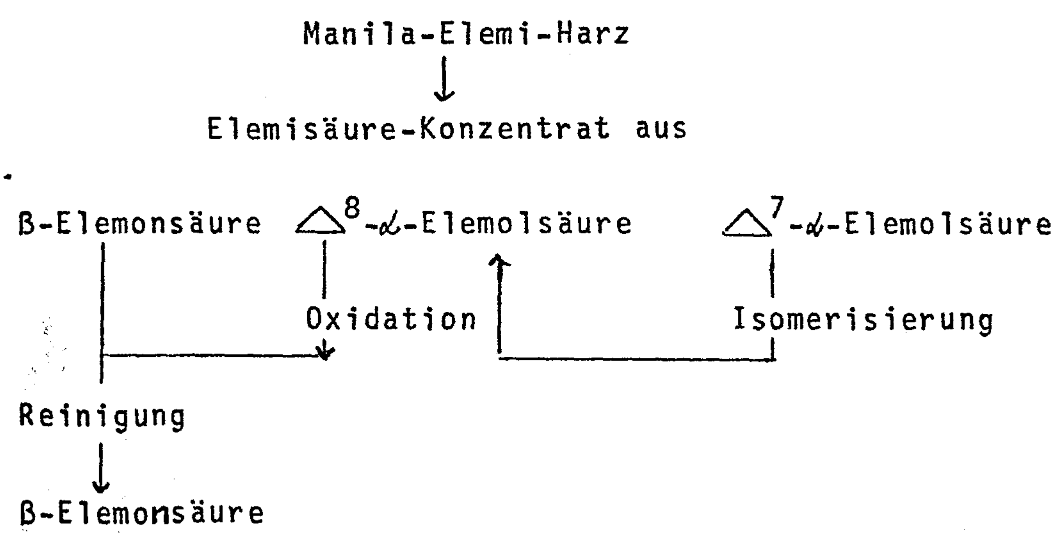

Die ß-Elemonsäure kann zu pharmazeutischen Präparaten verarbeitet werden, indem eine bestimmte Menge an Wirksubstanz im Gemisch mit anorganischen oder organischen, festen oder flüssigen, pharmazeutisch verwendbaren Trägerstoffen, die sich enteral oder parenteral verabreichen lassen, gemischt wird.

So verwendet man Tabletten oder Gelatinekapseln, welche den Wirkstoff zusammen mit Verdünnungsmitteln, wie z. B. Lactose, Dextrose, Sucrose, Mannit, Sorbit, Cellulose und/oder Glycerin und Schmiermittel, z.B. Kieselerde, Talk, Stearinsäure oder Salze davon, wie Magnesium oder Calciumstearat und/oder Polyethylenglykol aufweisen. Tabletten enthalten ebenfalls Bindemittel, z.B. Magnesium-, Aluminiumsilikat, Stärke, wie Mais-, Weizen-, Reisstärke, Gelatine, Methylcellulose, Natriumcarboxymethylcellulose und/oder Polyvinylpyrrolidon und, wenn erwünscht, Sprengmittel, z.B. Stärken, Agar, Alginsäure oder ein Salz davon, wie Natriumalginat, und/oder Brausemischungen oder Adsorptionsmittel, Farbstoffe, Geschmacksstoffe und Süßmittel. Ferner kann man die ß-Elemonsäure in Form von injizierbaren, z.B. intravenös verabreichbaren Präparaten oder Infusionslösungen einsetzen. Solche Lösungen sind vorzugsweise isotonische wäßrige Lösungen oder Suspensionen, wobei diese z.B. aus lyophilisierten Präparaten, welche die Wirksubstanz allein oder zusammen mit einem Trägermaterial enthalten, vor Gebrauch hergestellt werden können. Die pharmazeutischen Präparate können in an sich bekannter Weise, z.B, mittels konventioneller Misch-, Granulier-, Dragier-, Lösungs- oder Lyophilisierungsverfahren, hergestellt werden.

Die jeweils zu verabreichenden Dosierungen der pharmazeutischen Zubereitungen enthalten 10 - 500 mg ß-Elemonsäure, bevorzugt 10 - 200 mg, insbesondere 50 - 100 mg, pro Dosis und kann ein- oder mehrmals pro Tag verabreicht werden.

Die pharmakologische Wirksamkeit der ß-Elemonsäure wurde in den folgenden Modellen ermittelt.

1. Die Bestimmung der antiphlogistischen Wirksamkeit erfolgte nach dem Rattenpfotenödem-Test nach Hillebrecht (J. Hillebrecht, Arzneim. Forsch. 1954, Bd. 4, S. 607). Hierbei wurde an je einer Hinterpfote von Ratten im Gewicht von 120 g bis 160 g durch subplantare Gabe von Carragenin (0,5 %ig in NaCl-Lösung 0,9 %ig) in einer Menge von 0,1 ml Lösung je Pfote ein Ödem erzeugt. Nach Gabe der Testsubstanz, die in der Regel ein Volumen von 10 ml je kg Körpergewicht nicht überschreiten soll, wird das Volumen der Pfote in einem Überlauf ermittelt. Nach 3 Stunden wird der Endwert festgestellt. Je Dosis wird der Versuch mit 5 Versuchs- und 5 Kontrolltieren eines Geschlechtes durchgeführt und mit der gleichen Tierzahl des anderen Geschlechts wiederholt. Zur Auswertung wird die prozentuale Hemmung des Ödems gegenüber der Kontrollgruppe angegeben.

Tabelle ß-Elemonsäure

| Dosis (mg/kg p.o.) | 1 | 10 | 100 |
|---|---|---|---|
| Hemmeffekt (%) | - 7 | - 21 | - 33 |

Eine Vergleichsuntersuchung mit Phenylbutazon, einer bewährten entzündungshemmenden Substanz, ergab im Carragenin-Pfotenödem-Test an Ratten nach Hillebrecht erst bei höherer Dosierung Ödemreduktionen.

Tabelle Phenylbutazon

| Dosis (mg/kg p.o.) | 10 | 100 |
|---|---|---|
| Hemmeffekt (%) | ± 0 | - 41 |

2. Als weiteres Modell zur Testung der antiphlogistischen Wirksamkeit wurde das mit Cobra Venom Factor (CVF) induzierte Rattenpfotenödem verwendet. Da CVF eine Entzündung über eine Aktivierung des Complementsystems hervorruft, können hier hemmende Substanzen ein neuartiges Wirkprinzip für eine Anwendung bei entzündlichen Erkrankungen eröffnen.
Zur Ödemprovokation wurden an je einer Hinterpfote von Ratten im Gewicht von 120 - 160 g durch subplantare Injektion von CVF (10 Einheiten in 1 ml NaCl-Lösung 0,9%ig) in einer Menge von 0,1 ml Lösung je Pfote ein Ödem erzeugt. Nach Gabe der Testsubstanz in einem Volumen von 10 ml je kg Körpergewicht wird das Volumen der Pfote in einem Überlauf ermittelt. Nach 3 Stunden wird der Endwert festgestellt. Je Dosis wird der Versuch mit 5 Versuchs- und 5 Kontrolltieren eines Geschlechts durchgeführt und mit der gleichen Tierzahl des anderen Geschlechts wiederholt. Zur Auswertung wird die prozentuale Hemmung des Ödems gegenüber der Kontrollgruppe angegeben. Dabei wurden folgende Werte ermittelt:

Tabelle ß-Elemonsäure

| Dosis (mg/kg p.o.) | 1 | 10 | 100 |
|---|---|---|---|
| Hemmeffekt (%) | - 24 | - 32 | - 49 |

Eine Vergleichsprüfung mit Phenylbutazon ergab in diesem Testmodell schwächere Hemmwerte, so daß für ß-Elemonsäure Vorteile abzuleiten sind.

Tabelle Phenylbutazon

| Dosis (mg/kg p.o.) | 1 | 10 | 100 |
|---|---|---|---|
| Hemmeffekt (%) | +10 | -28 | - 34 |

3. Als weiteres relevantes Tiermodell zur Prüfung von Antirheumatika wurde die Adjuvans-Arthritis nach C. M. PEARSON, Proc. Soc. Exp. Biol. 91, 95-101 (1956) durchgeführt.
Pro Dosis werden 10 Wistar-Ratten im Gewicht von 120 - 150g eingesetzt. Die gleiche Tierzahl dient als Kontrolle. Mit 0,5 ml Freud'schem Adjuvans, subplantar injiziert, wird eine Arthritis erzeugt. Der Versuch dauert 17 Tage. Bei Versuchsbeginn wird das Pfotenvolumen der nicht-injizierten Hinterpfote ermittelt und dieses als Ausgangswert benutzt. Weitere Volumenmessungen werden am 14. und 17. Versuchstag durchgeführt. Bei der Auswertung wird die Differenz zwischen Ausgangs- und Endvolumen der Pfote, sowohl von der Versuchsgruppe als auch von der Kontrollgruppe gebildet und die Hemmung prozentual ausgedrückt.
Bei täglicher Gabe vom Zeitpunkt der Arthritis-Induktion an (prophylaktische Applikation) wurden folgende Hemmwerte (%) ermittelt:

| | ß-Elemonsäure | | | Phenylbutazon | | |
|---|---|---|---|---|---|---|
| Dosis (mg/kg p.o.) | 1 | 10 | 100 | 10 | 31,6 | 82,5 |
| 14. Tag p.i. | -55 | -62 | -165 | -42 | -57 | -58 |
| 17. Tag p.i. | -38 | -47 | - 92 | -31 | -51 | -43 |

Auch bei therapeutischer Applikation, beginnend 10 Tage nach Arthritisinduktion zeichneten sich starke Hemmwerte (%) von ß-Elemonsäure ab.

| | ß-Elemonsäure | | |
|---|---|---|---|
| Dosis (mg/kg p.o.) | 1 | 10 | 100 |
| 14. Tag p.i. | +36 | -13 | -38 |
| 17. Tag p.i. | -31 | -42 | -81 |

Die häufigste Nebenwirkung bei bekannten Antirheumatica ist eine schlechte gastrointestinale Verträglichkeit mit Ulcusinduktion. Deshalb wurde die ß-Elemonsäure auf Ulcusinduktion hin untersucht.

Die Bestimmung der Ulcusbildung erfolgte nach W.J.R.Whittle, Brit. J. Pharmacology 1975, Bd. 55, S. 242-43; L. Mariani, Europ. J. Toxicol. Eviron, 1975, Bd. 8, S. 335-39; R. Menguy und L. Desbaillets, Proc. Soc. Bio. Bd. 125, S. 1108.

Bei den Versuchen wurden je Dosis und Kontrolle 10 männliche Wistarratten (120 - 150 g, die 16 Stunden nüchtern gehalten wurden) verwendet. Die Provokation des blutigen Magengeschwüres erfolgte durch orale Applikation des Wirkstoffes. Nach 3,5 Stunden wurden die Tiere getötet, der Magen entnommen, längs der großen Kurvatur geöffnet und auf eine Styroporplatte gespannt. Ermittelt wird der mittlere Ulcusfaktor des Versuchs und der Kontrollgruppe. Dabei wurden bei einmaliger Gabe nach vorheriger Sensibilisierung der Magenmukosa durch eine 3tägige kohlenhydratreiche Kost folgende Effekte festgestellt:

| | ß-Elemonsäure | | | Phenylbutazon |
|---|---|---|---|---|
| Dosis (mg/kg p.o.) | 10 | 100 | 316 | 200 |
| Effekt | 0 | 0 | 0 | +++ |

0 = keine Ulcusinduktion
\+ = mäßige Ulcusinduktion
++ = starke Ulcusinduktion
+++ = sehr starke Ulcusinduktion

Die Bestimmung der Toxizität der ß-Elemonsäure erfolgte an der Maus und an der Ratte.
Akute perorale Toxizität an der Maus: > 4.640 mg/kg
Vergleich Phenylbutazon oral Maus : $LD_{50}$ 465 mg/kg

In einer dose-range-Studie an Ratten traten bei täglicher Applikation über 14 Tage mit Dosierungen von 681,0 und 1.000,0 mg/kg Körpergewicht keine gravierenden Nebenwirkungen auf.

Zur Bestimmung der Nebenwirkungen wurde ß-Elemonsäure mit Prednisolon verglichen.
Bekannte entzündungshemmende Substanzen können unerwünschte Nebenwirkungen verursachen, die teilweise bereits bei therapeutischen Dosen auftreten. Daher wurde für diese Fragestellung ß-Elemonsäure mit Prednisolon vergleichend untersucht (H.J. Lee, M.R.I. Soliman in Sciene 215, 989-991, 1982).
Die Applikation wurde an jungen Ratten vorgenommen, die Applikationsdauer betrug 8 Tage. ß-Elemonsäure wurde oral bis zu einer Dosierung von 316 mg/kg KGW verabreicht, Prednisolon intramuskulär bis zu einer Dosierung von 5 mg/kg KGW.
Im einzelnen wurden folgende Befunde erhoben:

| | ß-Elemonsäure | | Prednisolon | |
|---|---|---|---|---|
| Dosis (mg/kg) | 100 | 316 | 2,5 | 5 |
| Körpergewichtsentwicklung | (-) | (-) | - | - |
| Lebergewichte | 0 | 0 | 0 | 0 |
| Thymusgewichte | 0 | (-) | --- | --- |
| Blutbild | | | | |
| Lymphozyten | 0 | 0 | (-) | - |
| Leukozyten | 0 | 0 | 0 | - |

0 = kein Effekt
(-) = geringfügige Hemmung
- = deutliche Hemmung
--- = sehr starke Hemmung

Bereits nach Abschluß der Adjuvans-Arthritis bei 17tägiger Substanzgabe (prophylaktische Applikation) wurde festgestellt, daß die Nebennierengewichte nach ß-Elemonsäure-Gabe im Rahmen der Norm liegen.

Bewertung/Wirkung von Elemonsäure zu bekannten Substanzen. Eine quantitative Bewertung von ß-Elemonsäure im Vergleich zu bekannten antiphlogistischen Substanzen wird durch die Bestimmung der therapeutischen Breite ermöglicht, d.h. durch die Sicherheit, mit der ein Wirkstoff angewendet werden kann. Zum Vergleich dient Indometacin als stark wirksames, sowie Phenylbutazon als schwächer wirksames Antiphlogistikum, eine Gewichtung, die aus den jeweiligen $ED_{50}$-Werten des Arthritis-Versuches hervorgeht. Die therapeutische Breite wird 1. durch das Verhältnis zwischen der $LD_{50}$ und der $ED_{30}$ im CVF-Pfotenödem und 2. durch das Verhältnis zwischen dem Dosis-Schwellenwert der Magenunverträglichkeit und der $ED_{50}$ im Arthritis-Modell angegeben. Die $ED_{30}$- und $ED_{50}$-Werte wurden mit Hilfe der Regressionsgeraden ermittelt:

Tabelle: Dosis-Wirkungsbeziehungen und therapeutische Breite

| | ß-Elemonsäure | Phenylbutazon | Indometacin |
|---|---|---|---|
| 1. $ED_{30}$ (mg/kg p.o.) CVF-Ödem | 3,98 | 37,66 | 4,58 |
| 2. $ED_{50}$ (mg/kg p.o.) Adj.Arthritis prophyl. | 4,64 | 47,50 | 0,26 |
| 3. $LD_{50}$ (mg/kg p.o.) - Maus | > 4640,00 | 465,00 | 40,00 |
| 4. Schwellendosis (mg/kg p.o.) Magenschleimhautverträglichkeit akut | >316,00 | 25,00 | 5,62 |
| 5. therap. Index 1 3. / 1. | >1165,00 | 12,35 | 8.73 |
| 6. therap. Index 2 4. / 2. | > 68,00 | 0,53 | 21,62 |

Der therapeutische Index 1 und 2 als Maß für die therapeutische Breite liegt bei ß-Elemonsäure um ein Vielfaches höher als bei den Vergleichssubstanzen. Somit zeigt ß-Elemonsäure neben der guten antiphlogistischen Aktivität Vorteile eine verbesserten Sicherheit in der Anwendung gegenüber bekannten Antiphlogistika.

<u>Beispiel 1</u>

<u>ß-Elemonsäure</u>

a) Elemisäurekonzentrat

1 kg Elemiharz werden in 10 l Methanol verrührt und vom Ungelösten abfiltriert. Die klare Lösung wird mit 1 kg eines stark basischen Ionenaustauschers ($OH^-$-Form, mit Methanol gewaschen) versetzt und 3 Stunden gerührt. Das Austauscherharz wird abfiltriert und mit Methanol solange gewaschen, bis im Filtrat keine Nebenprodukte mehr nachgewiesen werden können. Die Überprüfung erfolgt mittels DC oder HPLC. Anschließend wird mit einer 10%igen methanolischen Essigsäurelösung eluiert, um die Elemisäuren vom Ionenaustauscher abzulösen. Mit weiterem reinen Methanol wird eluiert, bis im Ablauf keine Elemisäure mehr nachgewiesen werden kann.

Als Anionaustauscher werden verwendet: Dowex 1 x 8, Amberlite IRA 401, Amberlyst A 26.

Nach Eindampfen des Eluats erhält man ein Elemisäurekonzentrat, bestehend aus:

ß-Elemonsäure ca. 15 - 20 %
$\Delta^8$-α-Elemolsäure ca. 50 - 60 %
$\Delta^7$-α-Elemolsäure ca. 20 - 30 %

Ausbeute bezogen auf Manila-Elemiharz: 200 - 250 g
Gehalt an Elemisäuren: 90 - 95 %

b) Isomerisierung von $\Delta^{7}$-$\alpha$-Elemolsäure zur $\Delta^{8}$-$\alpha$-Elemolsäure im rohen Elemisäuregemisch.

1 kg Elemisäuregemisch (hergestellt nach Methode a) werden in 6 l Chloroform gelöst, mit 1 l 36%iger Salzsäure versetzt und 5 Stunden bei Raumtemperatur gerührt. Die Chloroformphase wird abgetrennt, mit Wasser und einer gesättigten Natriumhydrogencarbonat-Lösung neutral gewaschen, über wasserfreiem Natriumsulfat getrocknet und eingeengt. Der erhaltene Rückstand wird aus 3 l Methanol umkristallisiert.

Ausbeute (bezogen auf eingesetztes Konzentrat): 60 - 70%

Zusammensetzung: 10 - 15 % ß-Elemonsäure
80 - 85 % $\Delta^{8}$-$\alpha$-Elemolsäure
$<1$ % $\Delta^{7}$-$\alpha$-Elemolsäure
ca. 5 % Nebenprodukte

c) Oxydation der $\Delta^{8}$-$\alpha$-Elemolsäure im Elemisäuregemisch.

In einem 4 l Rundkolben mit Innenthermometer, Rührer und aufgesteckter Destillationsbrücke werden
200 g Elemisäuregemisch (hergestellt nach Methode b)
122 g Aluminium-isopropylat und
1500 ml Cyclohexanon
bei 60°C Badtemperatur gerührt bis eine klare Lösung entsteht. Anschließend wird die Badtemperatur auf 100°C gesteigert und der Ansatz weitere 5 - 6 Stunden gerührt, wobei ein schwaches Vakuum von 200 mbar angelegt wird, um gebildetes Cyclohexanol auszuschleppen. Überprüfung der Reaktion mittels DC bzw. HPLC. Nach Abkühlen auf Raumtemperatur wird der Ansatz in ein 5 l Becherglas überführt. Unter Rühren werden ca. 1 l Scherbeneis und 200 ml 36%iger Salzsäure zugegeben. Danach gibt man 1 l Wasser und 1 l Dichlormethan zu und rührt weitere 5 - 10 Minuten. Die organische Phase wird im Scheidetrichter abgetrennt und mit 2 l einer gesättigten Natriumhydrogencarbonat-Lösung zur Entfernung überschüssiger Säure verrührt. Die organische Phase wird mit Wasser neutralgewaschen und über wasserfreiem Natriumsulfat getrocknet.

Das Dichlormethan wird am Rotationsverdampfer bei einer Badtemperatur von 50°C abdestilliert und anschließend bei 100°C und Wasserstrahlpumpen-Vakuum das überschüssige Cyclohexanon entfernt. Der Rückstand wird mit gleichem Volumenanteil n-Hexan versetzt und 3 - 6 Stunden gerührt. Der weiße Feststoff wird abgenutscht, mit n-Hexan gewaschen und im Vakuumtrockenschrank getrocknet.
Ausbeute an roher ß-Elemonsäure (bezogen auf eingesetztes Elemikonzentrat: 53 - 60 %

d) Endreinigung der ß-Elemonsäure
Eine Chromatographiesäule (20 cm Ø, Länge 50 cm), wird mit 4 kg Kieselgel Si 60, 0,063-02 mm Korngröße, gefüllt und mit Dichlormethan durchfeuchtet. 400 g rohe ß-Elemonsäure (hergestellt nach Methode c)) werden in 2 l Dichlormethan heiß gelöst und nach Abkühlen auf die Säule gegeben. Mit einem Gemisch Dichlormethan/Methanol 100 : 1 wird eluiert. Als Vorlauf werden ca. 30 l Eluat aufgefangen. Aus dem Hauptlauf (50 - 70 l Eluat) wird die ß-Elemonsäure durch Einengen und anschließendem Umkristallisieren aus Aceton rein erhalten.
Der Verlauf der Chromatographie wird mittels DC bzw. HPLC überwacht.
Die Ausbeute an reiner ß-Elemonsäure beträgt 240 - 300 g entsprechend 60 - 75 %.

Beispiel 2

Herstellung von Tabletten.

Zusammensetzung:

| | |
|---|---|
| ß-Elemonsäure | 50,0 mg |
| Milchzucker | 38,0 mg |
| Maisstärke | 50,0 mg |
| Polyvinylpyrrolidon | 5,0 mg |
| Kolloidales Siliciumdioxid | 2,0 mg |
| Magnesiumsterarat | 1,0 mg |
| Natriumcarboximethylstärke | 4,0 mg |

Elemonsäure, Milchzucker und Maisstärke werden mit einer wäßrigen Lösung von Polyvinylpyrrolidon granuliert. Das getrocknete Granulat wird mit Magnesiumstearat, kolloidalem Siliciumdioxid und Natriumcarboximethylstärke gemischt und zu Tabletten mit einem Durchmesser von 8 mm verpreßt.

Beispiel 3

Herstellung von Hartgelatinekapseln.

Zusammensetzung:

| | |
|---|---|
| ß-Elemonsäure | 80,0 mg |
| Milchzucker | 50,0 mg |
| Maisstärke | 70,0 mg |
| Magnesiumstearat | 1,0 mg |
| kolloidales Siliciumdioxid | 2,0 mg |

Der Wirkstoff wird mit den Hilfsstoffen gemischt und in Hartgelatinekapseln der Größe 3 abgefüllt.

Anmelder: A. Nattermann & Cie GmbH (E-1653)
Nattermannallee 1, 5000 Köln 30

Titel: Verfahren zur Gewinnung von ß-Elemonsäure und diese enthaltende pharmazeutische Zubereitungen.

Patentansprüche

1. Verfahren zur Gewinnung von ß-Elemonsäure aus einer alkoholischen Elemi-Harz-Lösung, dadurch gekennzeichnet, daß man unter Anwendung von basischen Ionenaustauschern ein aus ß-Elemonsäure, $\Delta^{7}\alpha$-Elemolsäure und $\Delta^{8}\alpha$-Elemolsäure bestehendes Elemisäure-Gemisch gewinnt, dessen $\Delta^{7}\alpha$-Elemolsäure in einem chlorierten Kohlenwasserstoff unter Verwendung einer Mineralsäure zu $\Delta^{8}\alpha$-Elemolsäure isomerisiert, die $\Delta^{8}$-Elemolsäure des rohen Säuregemisches durch Oxydation in die ß-Elemonsäure überführt und durch Chromatographie über Kieselgel die reine ß-Elemonsäure abtrennt.

2. Pharmazeutische Zubereitungen mit üblichen Hilfs- und Trägerstoffen, dadurch gekennzeichnet, daß sie als wirksame Komponente ß-Elemonsäure enthalten, ausgenommen Elemiharz enthaltende pharmazeutische Zubereitungen.

3. Verwendung von pharmazeutischen Zubereitungen gemäß Anspruch 2 zur Bekämpfung von entzündlichen Prozessen.